# EUROPEAN PATENT SPECIFICATION

(11) **EP 1 481 691 B1**
(45) Date of publication and mention of the grant of the patent: **20.06.2007**
(21) Application number: 02765423.5
(22) Date of filing: 05.09.2002
(51) Int. Cl.: A61L 2/06, A61B 1/00, A61L 2/07, A61L 2/26, A61L 2/24

(54) **STERILIZER AND STERILIZING METHOD**
STERILISATOR UND STERILISATIONSVERFAHREN
STERILISATEUR ET PROCEDE DE STERILISATION

(30) Priority: 08.03.2002 JP 2002064140
(43) Date of publication of application: 01.12.2004
(73) Proprietor: Olympus Corporation, Tokyo 151-0072 (JP)
(72) Inventor: HASEGAWA, Hitoshi, Kohoku-ku, Yokohama-shi, Kanagawa 222-00 (JP); SUZUKI, Eiri, Sagamihara-shi, Kanagawa 229-0038 (JP); KUROSHIMA, Hisashi, 243 Olympus-Nishihachioji-Corp, Hachioji-shi, Tokyo 193-0832 (JP); NOGUCHI, Toshiaki, Tachikawa-shi, Tokyo 190-0002 (JP)
(74) Representative: von Hellfeld, Axel
(86) International application number: PCT/JP2002/009059
(87) International publication number: WO 2003/075962

(56) References cited:
- EP-A- 0 852 146
- JP-A- 5 337 082
- JP-A- 10 054 643
- JP-A- 2002 306 412
- JP-A- 2002 325 719
- JP-B2- 7 032 880
- JP-U- 7 039 714
- US-A- 3 368 865

## Description

### Technical Field

The present invention relates to a sterilizing apparatus comprising a chamber which houses an object to be sterilized so as to sterilize the object using high pressure and temperature steam.

### Background Art

Jpn. Pat. Appln. KOKAI Publication No. 11-56977 discloses a high pressure steam sterilizing apparatus in which a heat exchanger plate is provided on an inner surface of a door of a chamber via a spacer to constitute a heating medium channel and in which a heating medium is allowed to flow through the heating medium channel via a heating medium supply port and a heating medium exhaust port to adjust the temperature of the heat exchanger plate to an appropriate value in accordance with an operation, thus preventing operators from being burned.

Further, Jpn. Pat. Appln. KOKAI Publication No. 10-225506 discloses a high pressure steam sterilizing apparatus in which in addition to a sterilizing heater that generates steam during a drying step, a drying heater that dries an object to be sterilized is provided and in which simultaneously with the drying of the object by the drying heater, cooling means cools a part of the apparatus main body to promote evaporation of moisture from the object.

Furthermore, with the conventional high pressure steam sterilizing apparatuses, a user installs the sterilized object in the chamber via a large opening formed in the changer, before executing a sterilizing process.

The above-described Jpn. Pat. Appln. KOKAI Publication No. 11-56977 has a drawback in that the operability will be degraded because when the door of the apparatus is opened for cooling purpose, high temperature steam and gas will escape from the chamber. Jpn. Pat. Appln. KOKAI Publication No. 10-225506 has a structure in which when the proximity of the drain of the chamber is cooled for drying purpose, the inside of the chamber can also be cooled. This structure is not fitted for an apparatus in which the inside of the chamber is kept warm for next sterilizing step.

Further, with the conventional high pressure steam sterilizing apparatuses, when the door is opened to take out the object from the chamber after the sterilization is finished, the vapor and warm gas will be escaped outside of the chamber via the open door. Because of this, the chamber itself will be cooled, necessitating a pre-heating operation for next sterilization. Further, when installing an object in the chamber, one has to carry the object into the chamber, requiring much labor.

### Disclosure of Invention

The object of the present invention is to provide a sterilizing apparatus and a sterilizing method with improvement of maintaining the temperature of the chamber and with improved operability at the time of installing the object.

According to the first aspect of the present invention, there is provided a sterilizing apparatus comprising:
a chamber which houses an object to be sterilized so as to sterilize the object using high pressure and temperature steam;
an opening/closing section which opens and closes the chamber; and
an outflow inhibiting mechanism which operates when the opening/closing section is opened, to inhibit outflow of the steam from the chamber to an exterior, in connection with an operation for opening the opening/closing section.

According to the second aspect of the present invention, there is provided a sterilizing method comprising:
a step of setting an object to be sterilized in a chamber and closing a opening/closing section provided in the chamber;
a step of executing a sterilizing process by supplying high temperature and pressure steam to the object set in the chamber;
a step of opening the opening/closing section after the sterilizing step has been finished, to take out the object; and
a step of inhibiting outflow of the steam from the chamber to an exterior in connection with an opening operation of the closing section.

### Brief Description of Drawings

FIG. 1 is a view showing the configuration of a high pressure steam sterilizing apparatus according to a first embodiment of the present invention.
FIG. 2 is a block diagram showing the general configuration of the sterilizing apparatus.
FIG. 3 is a flow chart illustrating the procedure of a high pressure steam sterilizing process executed by the sterilizing apparatus.
FIG. 4 is a view illustrating means for reducing the temperature of a chamber opening.
FIG. 5 is a chart showing how the pressure in the chamber varies in accordance with the progress of a series of sterilizing steps as well as timings used to turn on and off or open and close components.
FIG. 6 is a view showing the appearance of a sterilizing apparatus 201 according to a second embodiment of the present invention.
FIG. 7 is a sectional view showing that in the sterilizing apparatus 201 according to the second embodiment, a door 204 is closed.
FIG. 8 is a sectional view showing that in the sterilizing apparatus 201 according to the second embodiment, the door 204 is open.
FIG. 9 is a view illustrating a variation of the second embodiment.
FIG. 10 is a view illustrating another variation of the second embodiment.

### Best Mode for Carrying Out the Invention

Embodiments of the present invention will be described below in detail with reference to the drawings.

### (First Embodiment)

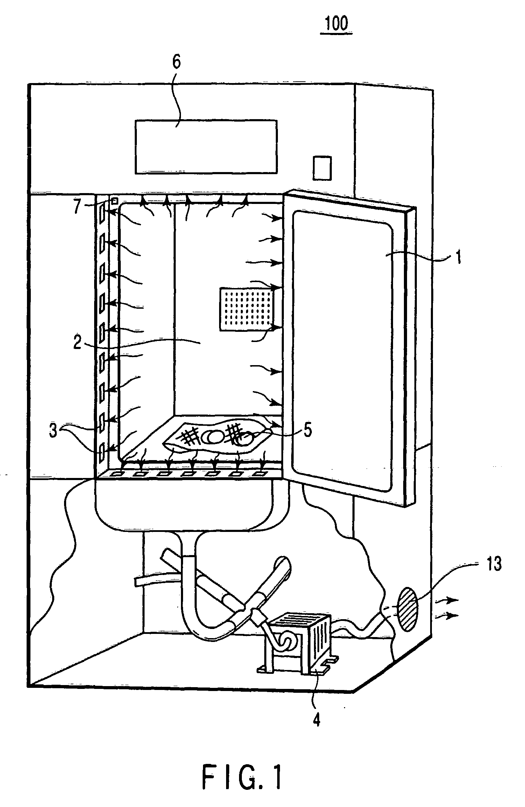

FIG. 1 is a view showing the configuration of a high pressure steam sterilizing apparatus (hereinafter referred to as a sterilizing apparatus) according to a first embodiment of the present invention. A chamber 2 is provided in an almost central portion of the sterilizing apparatus 100. The chamber 2 is used to house an object 5 to be sterilized so as to sterilize it using high pressure and temperature steam. A door (an opening/closing section) 1 is provided to close an opening in the chamber 2. A door sensor 7 senses when the door 1 is closed. A sterilizing process is executed by an operator by operating an operation panel 6 to given an instruction to the apparatus.

Further, a suction duct (outflow inhibiting mechanism) 3 is provided at an opening entry (in the vicinity of a periphery) of the chamber 2 to suck high temperature steam or gas. The suction duct 3 is connected to a suction pump 4 inside the apparatus. Reference numeral 13 denotes an exhaust duct.

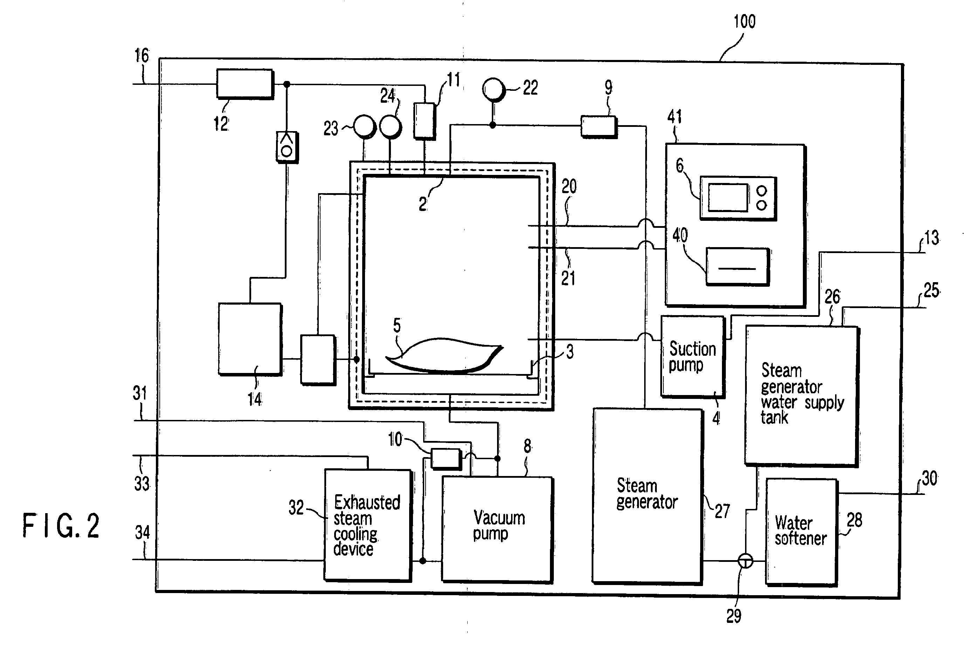

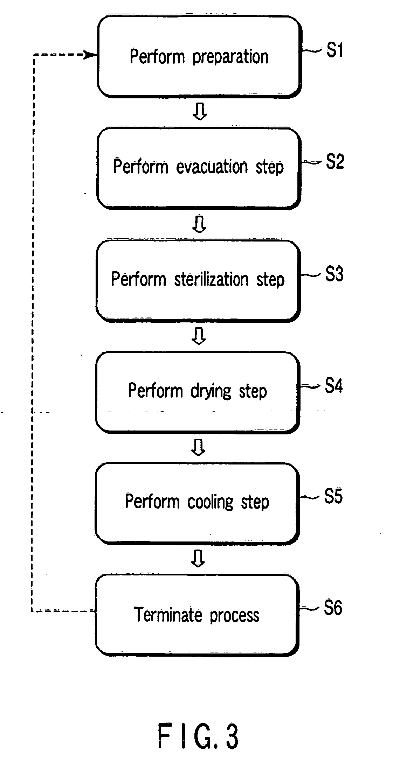

FIG. 2 is a block diagram showing the general configuration of the sterilizing apparatus 100. FIG. 3 is a flow chart illustrating the procedure of a high pressure steam sterilizing process executed by the sterilizing apparatus 100. With reference to FIGS. 1 to 3, description will be given of the high pressure steam sterilizing process according to the present invention. First, as a preparatory step (step S1), an operator opens the door 1, sets the object 5 contained in a sterilizing pack or the like, and closes the door 1. The operator confirms that the door 1 is closed and then pushes a switch on the operation panel 6 to start a sterilizing process. When the door sensor 7 confirms that the door 1 is closed, the door 1 of the apparatus is locked. Then, a series of sterilizing steps described below are started.

First, an evacuation step (step S2) is executed in which a vacuum pump 8 evacuates air from the chamber 2 to reduce the pressure in the chamber 2. Once the pressure in the chamber 2 decreases to a preset value (for example, -0.09 MPa), the vacuum pump 8 is stopped. Then, a steam supply valve 9 is opened to introduce high pressure steam generated by a steam generator 27 into the chamber 2. Thus, a sterilizing step (step S3) is started. Steam is fed into the chamber 2 until the temperature in the chamber reaches a preset sterilizing temperature (for example, 135°C). To improve efficiency, the vacuum evacuation and the steam supply may be alternated.

Once the temperature in the chamber 2 reaches the set sterilizing temperature, the interior of the chamber 2 is kept at this temperature for a preset sterilizing time (for example, 5 minutes). When the sterilizing time has passed, a steam discharge valve 10 is opened to discharge the high pressure steam from the chamber 2 to its exterior. Then, an exhausted steam cooling device 32 uses cooling water supplied through a cooling water supply port 33 to cool and convert the discharged high pressure steam into water. The water is then drained from a drain exhaust port 34.

After the steam has been discharged, a drying step (step S4) is executed in which the vacuum pump 8 reduces the pressure in the chamber 2. This reduces the pressure in the chamber 2 while maintaining the high temperature. Consequently, water droplets are rapidly vaporized and dried.

When a preset drying time (for example, 10 minutes) has passed, the vacuum pump 8 is stopped. Then, an intake valve 11 is opened to return the pressure in the chamber 2 to atmospheric pressure. To prevent the interior of the chamber 2 from being contaminated by indigenous bacteria in the air, a filter called a sterile filter 12 and having a filter mesh of at most 0.2 microns is attached to an air intake port 16 of the intake valve 11.

Once the pressure in the chamber 2 has returned to atmospheric pressure, the door 1 is unlocked. The operator confirms that the door 1 has been unlocked and then opens the door 1. At this time, the door sensor 7 senses the opening to start the suction pump 4 to execute a cooling step (step S5). Further, since the temperature in the chamber 2 is high, remaining vapor or steam is sucked through the suction duct 3 in the chamber opening. The vapor passes through the suction pump 4 and is then discharged from the exhaust duct 13, located in the rear of a side of the apparatus, to the exterior of the apparatus.

When a preset specified time has passed or the temperature of the atmosphere, measured by a temperature sensor 20 provided in the chamber 2 or near the opening in the chamber 2, becomes equal to or lower than a set temperature, the suction pump 4 is stopped. This minimizes a decrease in the temperature in the chamber 2 in order to maintain a preheating effect for the next sterilizing operation. Thus, a series of sterilizing steps are completed (step S6). The operator can then set the next object to be sterilized in the chamber 2.

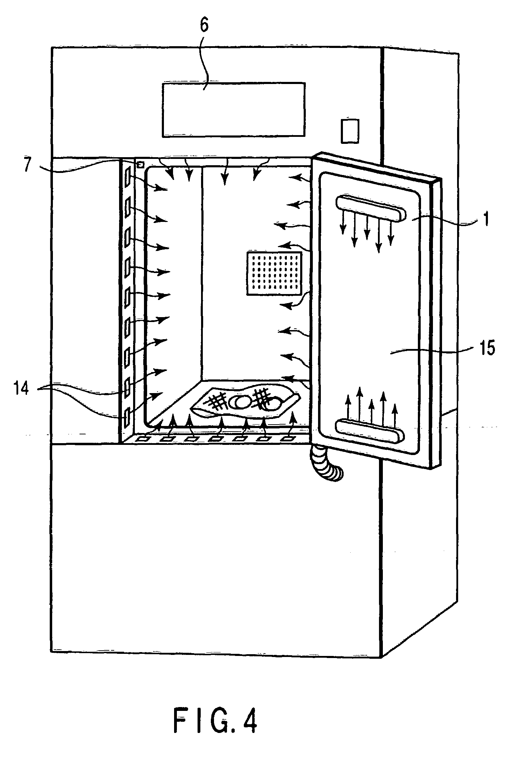

As means for reducing the temperature in the chamber opening, the door sensor 7 may sense when the door 1 is opened as shown in FIG. 4, so that cooling air may shoot from a cooling duct 14 provided around the periphery of the chamber opening or inside the door 1. Alternatively, as shown in FIG. 4, heat-sensitive paint 15 or a seal like object which has its color changed by the temperature may be attached to the inside of the door 1 or to the chamber opening so as to allow the operator to visually confirm that the temperature has sufficiently decreased.

In FIG. 2, reference numerals 21, 22, 23, and 24 denote pressure gauges. The operation panel, together with a recording device 40, constitutes a control box 41. A steam generator water supply tank 26 connected to a manual water supply port 25 and a water softener 28 connected to a steam water supply port 30 are connected the steam generator 27 via a change-over valve 29.

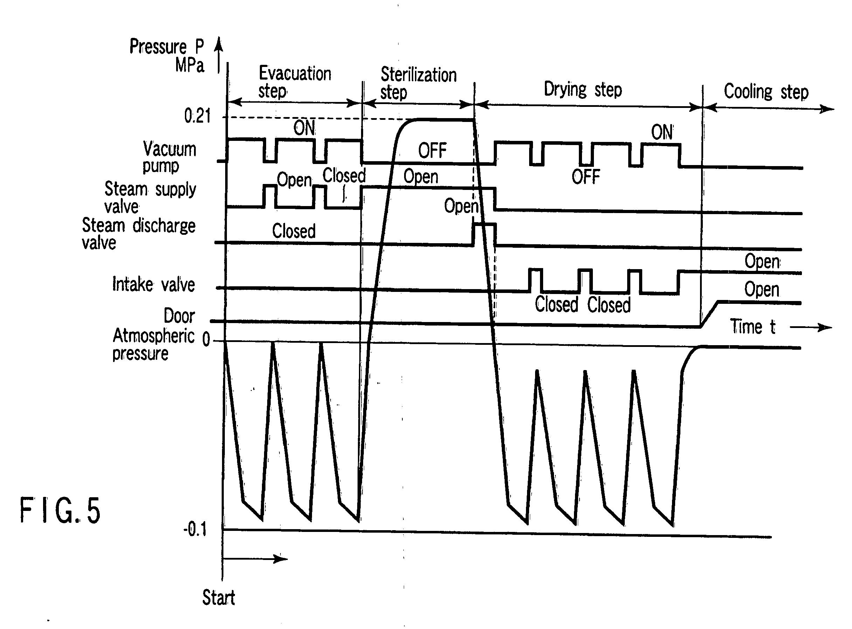

A time chart in FIG. 5 shows how the pressure in the chamber 2 varies in accordance with the progress of the series of sterilizing steps as well as timings used to turn on and off or open and close the components. During an ON period of the vacuum pump 8, the pump 8 is repeatedly turned on and off. The timings for turn on and off correspond to the waveform of atmospheric pressure.

According to the first embodiment, in a high pressure steam sterilizing apparatus that sterilizes an object using high temperature steam or gas, after sterilization, the suction pump 4 or a fan is operated at almost the same time when the door 1, which closes the chamber 2, is opened. Further, the suction duct 3, provided around the periphery of the opening in the chamber 2, sucks and discharges high temperature steam and gas to the exterior of the apparatus. This improves operability.

### (Second Embodiment)

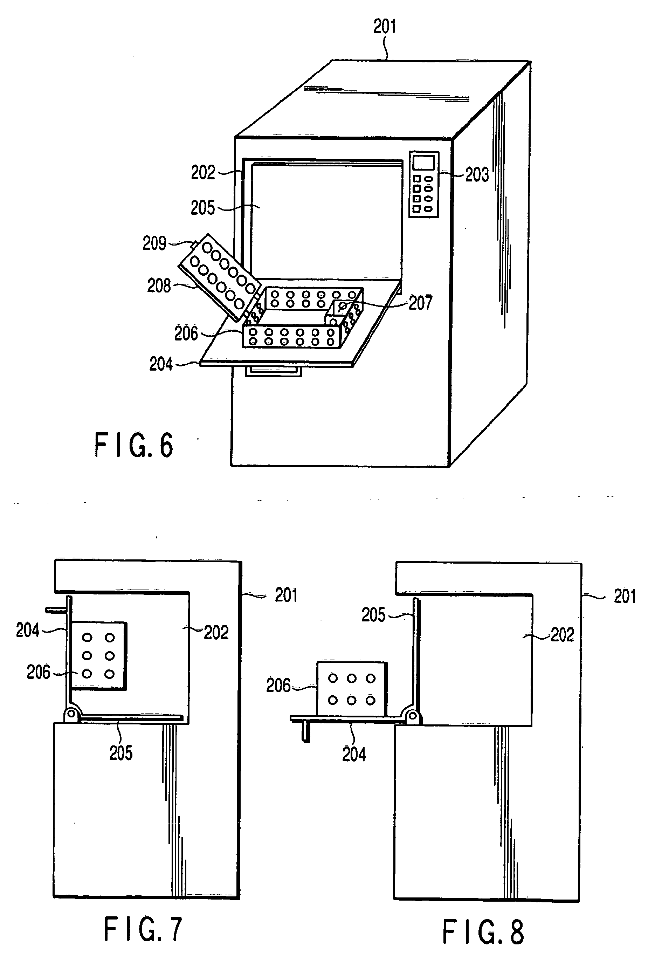

Now, a second embodiment will be described with reference to FIGS. 6 to 8. FIG. 6 shows the appearance of a sterilizing apparatus 201 according to the second embodiment. When an object is housed in a chamber 202 of the sterilizing apparatus 201, a sterilizing process is executed using high pressure steam. Further, the sterilizing apparatus 201 is provided with an operation panel 203. The sterilizing apparatus 201 is operated by operating the operation panel 203. The sterilizing apparatus 201 is provided with a door 204 so as to open and close the chamber 202. A screen 205 is formed integrally with the door 204. when the door 204 is opened, the screen (outflow inhibiting mechanism) 205 shields a chamber opening.

Further, the door 204 is opened until its horizontal position. When the door 204 is opened, a housing case 206 appears which is fixed to the inside of the door 204 to house an object. The housing case 206 is provided with an indicator used to check whether or not the object has been reliably sterilized and an indicator installation space 207 used to install the indicator or a test pack.

Moreover, the housing case 206 is provided with a opening/closing cover 208 with a lock portion 209. Further, the housing case 206, its opening/closing cover 208, and the indicator installation space 207 are provided with a large number of vent holes to allow steam to permeate into the housing case 296 during a sterilizing process.

FIG. 7 is a sectional view showing the sterilizing apparatus 201 according to the present embodiment, in which the door 204 is closed. FIG. 8 is a sectional view showing the sterilizing apparatus 201, in which the door 204 is open. The door 204 and the screen 205 are formed integrally with each other so as to form a right angle between them. Thus, in the state shown in FIG. 7, when the door 204 is opened until its horizontal position, the screen 205 stands up to its vertical position to close the opening in the chamber 202 (FIG. 8).

Now, description will be given of the operation of the second embodiment of the present invention. When an object is to be sterilized, the door 204 is first opened to draw out the housing case 206. At this time, the screen 205, integrated with the door 204, closes the opening in the chamber 202. It is thus possible to avoid allowing steam or vapor in the chamber 202 to leak out to prevent the user's operations or reducing the temperature in the chamber 202.

Then, the door 204 is opened until its horizontal position to draw out the housing case 206. Then, the opening/closing cover 208 is opened and the object is installed in the housing case 206. Further, at this time, the indicator, used to check whether or not the object has been reliably sterilized, and a test pack are also installed in the indicator installation space 207. Then, finally, the opening/closing cover 208 is closed and is locked using the lock portion 209.

In this manner, the object can be installed from the exterior of the chamber 202. This eliminates the need to access the interior of the chamber 202 to allow the object to be easily installed. Consequently, the user can perform operations more easily and efficiently. Then, after the object has been housed in the housing case 206, the door 204 is closed to place the housing case 206 in the chamber 202 to load the object into the chamber 202.

After the door 204 has been closed, the operation panel 203 is operated to execute a sterilizing process. When the sterilizing process is started in the sterilizing apparatus 201, sterilizing high temperature vapor is supplied into the chamber 202. Then, the vapor permeates into the housing case 206 through the vent holes in the housing case 206 to come into contact with the object for sterilization.

Once a predetermined sterilizing process has been finished, the operation panel 203 shows that the process has been finished. The user confirms this display and then opens the door 204 to take out the object. In this case, the door 204 and the screen 205 are formed integrally with each other so as to have a right angle between them. Accordingly, when the door 204 is opened until the horizontal position, the screen 205 stands up to its vertical position to close the opening in the chamber 202. This prevents the steam in the chamber 202 from leaking out.

This configuration can prevent the temperature of the chamber 202 from decreasing. Thus, during subsequent sterilizing processes, it is possible to shorten a preheating step of preheating the chamber 202. Then, the housing case 206 is drawn out toward the user by opening the door 204. The lock portion 209 is then released to open the opening/closing door 208. The sterilized object is then taken out.

In this manner, the sterilized object can be loaded into and taken out of the chamber 202 by opening and closing the door 204. Accordingly, the operator can easily perform a sterilizing operation without accessing the interior of the chamber 202.

In the above embodiment, the screen 205 is integrated with the door 204. However, the screen 205 and the door 204 may be formed separately so that when the door 204 is opened, a motor is used to automatically raise the screen 205 from the bottom of the chamber 202. Of course, conversely, the door 204 may be lowered from above.

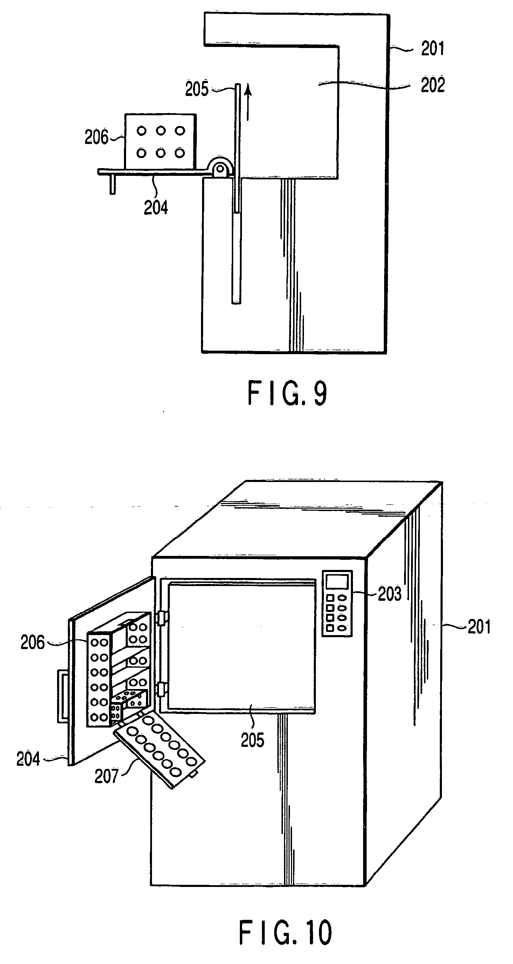

Alternatively, the door 204 may be opened and closed in a lateral direction as shown in FIG. 10 rather than toward the user as in the case of the above embodiment. Further, the housing case 206 may have a mesh structure rather than being provided with the large number of vent holes as in the case of the above embodiment.

According to the second embodiment, the object to be sterilized is housed and taken out by opening and closing the chamber opening/closing mechanism of the sterilizing apparatus. Consequently, the user need not access the interior of the chamber and can thus perform operations more easily and efficiently. Further, when the opening/closing means is opened, the opening in the chamber is closed. This prevents a decrease in the temperature of the chamber 202 to shorten the preheating step.

### Industrial Applicability

According to the present invention, after sterilization, when the door that closes the chamber is opened, high temperature steam or gas remaining in the chamber is sucked through the sunction duct and discharged to the exterior of the apparatus. This improves operability.

Further, according to the present invention, the sterilized object is housed and taken out by opening and closing the chamber opening/closing mechanism of the sterilizing apparatus. Consequently, the user need not access the interior of the chamber and can thus perform operations more easily and efficiently. Furthermore, when the opening/closing means is opened, the opening in the chamber is closed. This serves to maintain the temperature of the chamber to shorten the preheating step.

## Claims

1. A sterilizing apparatus (100; 201) comprising:
a chamber (2; 202) which houses an object to be sterilized so as to sterilize the object using high pressure and temperature steam;
an opening/closing section (1; 204) which opens and closes the chamber (2; 202); and
**characterized by** an outflow inhibiting mechanism (3; 205) which operates when the opening/closing section is opened, to inhibit outflow of the steam from the chamber (2; 202) to an exterior through the opening/closing section (1; 204) in connection with an operation for opening the opening/closing section.

2. The sterilizing apparatus (100; 201) according to claim 1, wherein the outflow inhibiting mechanism (3; 205) includes a sucking section (3) provided in the vicinity of a periphery of an opening through which the object is housed in or taken out of the chamber (2; 202), and sucks the steam via the sucking section (3) in connection with the operation for opening the opening/closing section (1; 204).

3. The sterilizing apparatus (100; 201) according to claim 1, wherein the outflow inhibiting mechanism (3; 205) includes a shielding member (205) which shields the opening through which the object is housed in or taken out of the chamber (2; 202), in connection with the operation for opening the opening/closing section (1; 204).

4. The sterilizing apparatus (100; 201) according to claim 3, wherein the shielding member (205) is integrated with the opening/closing section (1; 204) and moves to a position where the shielding member completely shields the opening when the opening/closing section is opened until the opening/closing section reaches an open position outside the apparatus.

5. The sterilizing apparatus (100; 201) according to claim 4, wherein a housing section (206) in which the object is housed is provided behind the opening/closing section in a state of being closed, and when the opening/closing section is opened until the opening/closing section reaches the open position outside the apparatus, the housing section (206) is placed at a position where the housing section can house the object.

6. The sterilizing apparatus (100; 201) according to claim 5, wherein the housing section (206) is provided with an indicator installation space (207) in which an indicator used to check whether or not the object has been reliably sterilized and others are installed.

7. The sterilizing apparatus (100; 201) according to claim 6, wherein the indicator installation space (207) is provided with a plurality of vent holes into which steam permeates during a sterilizing process.

8. The sterilizing apparatus (100; 201) according to claim 6, wherein the indicator installation space (207) has a mesh structure into which steam permeates during a sterilizing process.

9. The sterilizing apparatus (100; 201) according to claim 3, wherein the shielding member (205) is provided separately from the opening/closing section (1; 204) and moves to a position where the shielding member completely shields the opening when the opening/closing section is opened until the opening/closing section reaches an open position outside the apparatus.

10. A sterilizing method comprising:
a step of setting an object to be sterilized in a chamber (2; 202) and closing an opening/closing section (1; 204) provided in the chamber;
a step of executing a sterilizing process by supplying high temperature and pressure steam to the object set in the chamber;
a step of opening the opening/closing section after the sterilizing step has been finished, to take out the object; and
**characterized by** a step of inhibiting outflow of the steam from the chamber to an exterior through the opening/closing section in connection with an opening operation of the opening/closing section.

## Patentansprüche

1. Sterilisierapparat (100; 201), der umfasst:
eine Kammer (2; 202) zur Aufnahme eines zu sterilisierenden Gegenstands, in der der Gegenstand unter Verwendung von Dampf hohen Drucks und hoher Temperatur sterilisiert wird, und
ein Öffnungs- und Schließteil (1; 204), das die Kammer (2; 202) öffnet und verschließt,
**gekennzeichnet durch** einen Sperrmechanismus (3; 205) gegen Ausströmen, der beim Öffnen des Öffnungs- und Schließteils wirksam wird, um das Ausströmen des Dampfs aus der Kammer (2; 202) nach außen **durch** das Öffnungs- und Schließteil (1; 204) in Verbindung mit einer Betätigung des Öffnungs- und Schließteils zum Öffnen zu verhindern.

2. Sterilisierapparat (100; 201) nach Anspruch 1, bei dem der Sperrmechanismus (3; 205) gegen Ausströmen einen Ansaugabschnitt (3) umfasst, der in der Nähe des Umfangs der Öffnung, durch welche der Gegenstand in die Kammer (2; 202) eingegeben oder aus ihr entnommen wird, vorgesehen ist und den Dampf durch den Absaugabschnitt (3) in Verbindung mit der Betätigung des Öffnungs- und Schließteil (1; 204) zum Öffnen absaugt.

3. Sterilisierapparat (100; 201) nach Anspruch 1, bei dem der Sperrmechanismus (3; 205) gegen Ausströmen ein Schild (205) aufweist, das die Öffnung, durch die das Objekt in die Kammer (2; 202) eingegeben oder entnommen wird, in Verbindung mit der Betätigung des Öffnungs- und Schließteils (1; 204) zum Öffnen absperrt.

4. Sterilisierapparat (100; 201) nach Anspruch 3, bei dem das Schild (205) in das Öffnungs- und Schließteil (1; 204) integriert ist und sich in eine Stellung bewegt, in der das Schild die Öffnung, wenn das Öffnungs- und Schließteil geöffnet wird bis das Öffnungs- und Schließteil eine Öffnungsstellung außerhalb des Apparats erreicht hat, vollständig absperrt.

5. Sterilisierapparat (100; 201) nach Anspruch 4, bei dem ein Kammerabschnitt (206), in den der Gegenstand eingegeben wird, hinter dem Öffnungs- und Schließsteil in einem verschlossenen Zustand vorgesehen ist, und bei dem der Kammerabschnitt (206), wenn der Öffnungs- und Schließteil geöffnet wird bis das Öffnungs- und Schließteil seine Öffnungsstellung außerhalb des Apparats erreicht hat, in einer Stellung angeordnet ist, in der der Kammerabschnitt den Gegenstand aufnehmen kann.

6. Sterilisierapparat (100; 201) nach Anspruch 5, bei dem der Kammerabschnitt (206) mit einem Indikatorinstallationsraum (207) versehen ist, in welchem ein Indikator zur Prüfung ob oder ob nicht der Gegenstand zuverlässig sterilisiert worden ist und andere eingebaut sind.

7. Sterilisierapparat (100; 201) nach Anspruch 6, bei dem der Indikatorinstallationsraum (207) mit mehreren Belüftungsöffnungen versehen ist, durch die Dampf während eines Sterilisiervorgangs eintritt.

8. Sterilisierapparat (100; 201) nach Anspruch 6, bei dem der Indikatorinstallationsraum (207) einen maschenartigen Aufbau hat, durch den Dampf während eines Sterilisiervorgangs eintritt.

9. Sterilisierapparat (100; 201) nach Anspruch 3, bei dem das Schild (205) getrennt von dem Öffnungs- und Schließteil (1; 204) vorgesehen ist und sich in eine Stellung bewegt, in der das Schild die Öffnung vollständig abschließt, wenn das Öffnungs- und Schließteil geöffnet wird bis das Öffnungs- und Schließteil seine Öffnungsstellung außerhalb des Apparats erreicht hat.

10. Sterilisierverfahren, das umfasst:
einen Schritt des Eingebens eines zu sterilisierenden Gegenstands in eine Kammer (2; 202) und des Schließens eines Öffnungs- und Schließteils (1; 204), das in der Kammer vorgesehen ist;
einen Schritt der Durchführung eines Sterilisiervorgangs durch Einführen von Dampf, hoher Temperatur und hohen Drucks an den in die Kammer eingegebenen Gegenstand;
einen Schritt des Öffnens des Öffnungs- und Schließteils, nachdem der Sterilisierschritt beendet worden ist, um den Gegenstand herauszunehmen, und
**gekennzeichnet durch** einen Schritt des Verhinderns des Ausströmens des Dampfes aus der Kammer nach außen **durch** den Öffnungs- und Schließteil in Verbindung mit einem Öffnungsvorgang des Öffnungs- und Schließteils.

## Revendications

1. Appareil (100 ; 201) de stérilisation comprenant :
une chambre (2 ; 202) qui abrite un objet à stériliser dans le but de stériliser l'objet à l'aide de vapeur à haute pression et haute température ;
une section (1 ; 204) d'ouverture/fermeture qui ouvre et ferme la chambre (2 ; 202) ; et
**caractérisé par** un mécanisme (3 ; 205) d'inhibition d'écoulement de sortie qui fonctionne quand la section d'ouverture/fermeture est ouverte, afin d'inhiber l'écoulement de sortie de la vapeur de la chambre (2 ; 202) vers l'extérieur par l'intermédiaire de la section (1 ; 204) d'ouverture/fermeture, en liaison avec un fonctionnement d'ouverture de la section d'ouverture/fermeture.

2. Appareil (100 ; 201) de stérilisation selon la revendication 1, dans lequel le mécanisme (3 ; 205) d'inhibition d'écoulement de sortie inclut une section (3) d'aspiration placée dans le voisinage de la périphérie d'une ouverture par laquelle l'objet est placé dans la chambre (2 ; 202) ou retiré de celle-ci, et qui aspire la vapeur par l'intermédiaire de la section (3) d'aspiration en liaison avec le fonctionnement d'ouverture de la section (1 ; 204) d'ouverture/fermeture.

3. Appareil (100 ; 201) de stérilisation selon la revendication 1, dans lequel le mécanisme (3 ; 205) d'inhibition d'écoulement de sortie inclut un élément (205) de protection qui protège l'ouverture par laquelle l'objet est placé dans la chambre (2 ; 202) ou retiré de celle-ci, en liaison avec le fonctionnement d'ouverture de la section (1 ; 204) d'ouverture/fermeture.

4. Appareil (100 ; 201) de stérilisation selon la revendication 3, dans lequel l'élément (205) de protection est intégré dans la section (1 ; 204) d'ouverture/fermeture et se déplace jusqu'à une position dans laquelle l'élément de protection protège complètement l'ouverture quand la section d'ouverture/fermeture est ouverte jusqu'à ce que la section d'ouverture/fermeture atteigne une position ouverte à l'extérieur de l'appareil.

5. Appareil (100 ; 201) de stérilisation selon la revendication 4, dans lequel une section (206) de logement dans laquelle l'objet est mis, est placée derrière la section d'ouverture/fermeture dans un état de fermeture, et quand la section d'ouverture/fermeture est ouverte jusqu'à ce que la section d'ouverture/fermeture atteigne la position ouverte à l'extérieur de l'appareil, la section (206) de logement est placée dans une position dans laquelle la section de logement peut abriter l'objet.

6. Appareil (100 ; 201) de stérilisation selon la revendication 5, dans lequel la section (206) de logement est munie d'un espace (207) d'installation d'indicateur dans lequel est installé un indicateur utilisé pour vérifier si l'objet a été ou non stérilisé de façon fiable ainsi que pour procéder à d'autres vérifications.

7. Appareil (100 ; 201) de stérilisation selon la revendication 6, dans lequel l'espace (207) d'installation d'indicateur est muni d'une pluralité de trous d'aération dans lesquels la vapeur s'infiltre pendant un processus de stérilisation.

8. Appareil (100 ; 201) de stérilisation selon la revendication 6, dans lequel l'espace (207) d'installation d'indicateur possède une structure de maillage dans lesquels la vapeur s'infiltre pendant un processus de stérilisation.

9. Appareil (100 ; 201) de stérilisation selon la revendication 3, dans lequel l'élément (205) de protection est fourni séparément de la section (1 ; 204) d'ouverture/fermeture et se déplace jusqu'à une position dans laquelle l'élément de protection protège complètement l'ouverture quand la section d'ouverture/fermeture est ouverte jusqu'à ce que la section d'ouverture/fermeture atteigne une position ouverte à l'extérieur de l'appareil.

10. Procédé de stérilisation comprenant :
une étape consistant à placer un objet à stériliser dans une chambre (2 ; 202) et à fermer une section (1 ; 204) d'ouverture/fermeture placée dans la chambre ;
une étape consistant à exécuter un processus de stérilisation par délivrance de vapeur à haute température et haute pression à l'objet placé dans la chambre ;
une étape consistant à ouvrir la section d'ouverture/fermeture après que l'étape de stérilisation ait été achevée, afin d'extraire l'objet ; et
**caractérisé par** une étape consistant à inhiber l'écoulement de sortie de la vapeur de la chambre vers l'extérieur par l'intermédiaire de la section d'ouverture/fermeture en liaison avec un fonctionnement d'ouverture de la section d'ouverture/fermeture.
